(19) Europäisches Patentamt
European Patent Office
Office européen des brevets

(11) Veröffentlichungsnummer: **0 287 907 B1**

# (12) EUROPÄISCHE PATENTSCHRIFT

(45) Veröffentlichungstag der Patentschrift: **19.08.92**

(51) Int. Cl.$^5$: **C07D 487/04**, A61K 31/505, //(C07D487/04,239:00,231:00)

(21) Anmeldenummer: **88105595.8**

(22) Anmeldetag: **08.04.88**

Die Akte enthält technische Angaben, die nach dem Eingang der Anmeldung eingereicht wurden und die nicht in dieser Patentschrift enthalten sind.

## (54) Pyrazolo[3,4-d]pyrimidine, Verfahren zu ihrer Herstellung und Verwendung als Arzneimittel.

(30) Priorität: **15.04.87 DE 3712735**

(43) Veröffentlichungstag der Anmeldung:
**26.10.88 Patentblatt 88/43**

(45) Bekanntmachung des Hinweises auf die Patenterteilung:
**19.08.92 Patentblatt 92/34**

(84) Benannte Vertragsstaaten:
**AT BE CH DE ES FR GB GR IT LI LU NL SE**

(56) Entgegenhaltungen:
**DE-B- 1 106 331**
**US-A- 3 399 196**

**JOURNAL OF MEDICINAL AND PHARMACEUTICAL CHEMISTRY, Band 5, 1962 E. Y. Sutcliffe et al. "Potential Purine Antagonists XXXII. The Synthesis and Antitumor Activity of Certain Compounds Related to 4-Aminopyrazolo(3,4-d)pyrimidine" Seiten 588-607**

(73) Patentinhaber: **BOEHRINGER MANNHEIM GMBH**
**Sandhofer Strasse 116**
**W-6800 Mannheim 31(DE)**

(72) Erfinder: **Friebe, Walter-Gunar, Dr.rer.nat**
**Sophienstrasse 8**
**W-6800 Mannheim(DE)**
Erfinder: **Kampe, Wolfgang, Dr.rer.nat**
**Ahornstrasse 42**
**W-6805 Heddesheim(DE)**
Erfinder: **Wilhelms, Otto-Henning, Dr.rer.nat.**
**Odenwaldstrasse 25/2**
**W-6941 Weinheim-Tittenweier(DE)**

EP 0 287 907 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Pyrazolo[3,4-d]pyrimidine der allgemeinen Formel I

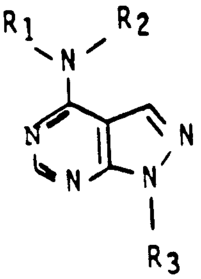

(I),

in welcher

- $R_1$ einen $C_1$- bis $C_6$-Alkylrest, einen $C_2$- bis $C_6$-Alkenylrest, einen $C_3$- bis $C_7$-Cycloalkylrest oder einen Arylrest,
- $R_2$ einen $C_2$- bis $C_6$-Alkenylrest, einen $C_3$- bis $C_7$-Cycloalkylrest oder einen gewünschtenfalls ein- oder mehrfach durch Halogen, $C_1$- bis $C_6$-Alkyl, Hydroxy, $C_1$- bis $C_6$-Alkoxy, $C_1$- bis $C_3$-Halogenalkyl, $C_3$- bis $C_7$-Alkoxycarbonyl, Aminocarbonyl, $C_2$- bis $C_7$-Alkylaminocarbonyl, $C_3$- bis $C_{13}$-Dialkylaminocarbonyl, Cyano oder $C_1$- bis $C_6$-Alkylthio substituierten Aralkyl-, Furylalkyl-, Thienylalkyl- oder Pyridinylalkylrest mit 1 bis 6 Kohlenstoffatomen im Alkylteil und
- $R_3$ Wasserstoff, einen gewünschtenfalls ein- oder mehrfach durch Hydroxy substituierten $C_2$- bis $C_6$-Alkylrest, einen Tetrahydrofuranylrest oder einen Tetrahydropyranylrest

bedeuten, mit der Maßgabe, daß $R_2$ nicht unsubstituiertes Benzyl sein kann, wenn $R_1$ für die Methylgruppe steht,
deren physiologisch verträgliche Salze anorganischer oder organischer Säuren und Verfahren zu ihrer Herstellung sowie diese Verbindungen enthaltende Arzneimittel.

Bekannt ist aus J. Med. Chem. Vol. 5, S. 588 (1962) 4-[N-(Benzyl)-methylamino]-1H-pyrazolo[3,4-d]-pyrimidin als potentielles Antitumormittel.

Für den Fall, daß die Verbindungen der allgemeinen Formel I ein asymmetrisches Kohlenstoffatom enthalten, sind auch die optisch aktiven Verbindungen und racemischen Gemische Gegenstand der Erfindung.

Die neuen Verbindungen der allgemeinen Formel I weisen wertvolle pharmakologische Eigenschaften auf, insbesondere können sie die antigenbedingte Freisetzung von Mediatoren aus Lungengewebeproben sowie die Kontraktion von Lungengewebestreifen hemmen. Sie eignen sich daher zur Behandlung allergischer Krankheiten sowie von entzündungsbedingten bronchospastischen und bronchokonstriktorischen Reaktionen.

Die Alkylreste in den genannten Gruppen sowie die Alkenyl-, die Alkoxy-, Alkylamino- und Alkylthioreste können geradkettig oder verzweigt sein. Bevorzugte Alkylreste sind der Methyl-, Ethyl-, n-Propyl-, Isopropyl-, n-Butyl-, Isobutyl-, n-Pentyl- und 3-Pentylrest. Ein Alkenylrest ist insbesondere der Allylrest. Alkoxy- und Alkylthioreste sind vorzugsweise der Methoxy-, Ethoxy-, Methylthio- und Ethylthiorest.

Cycloalkylreste sind insbesondere Cyclopentyl und Cyclohexyl.

Die Furylalkyl-, Thienylalkyl- und Pyridinylalkylreste sind bevorzugt der Furfuryl-, Thenyl- und Pyridinylmethyl-Rest.

Als Aralkylreste kommen beispielsweise der Benzyl-, Phenethyl-, Phenylpropyl-, Phenylisopropyl- oder 3-Methyl-3-phenylpropylrest in Frage.

Aryl bedeutet in der Regel Naphthyl oder Phenyl, bevorzugt ist der Phenylrest.

Halogenatome sind insbesondere Fluor, Chlor, Brom und Jod.

Für den Rest $R_3$ können insbesondere folgende Gruppen stehen: Wasserstoff, 2-Hydroxyethyl, 2-Hydroxypropyl, 3-Hydroxypropyl, 2,3-Dihydroxypropyl, Tetrahydrofuran-2-yl und Tetrahydropyran-2-yl.

Außer den in den Beispielen genannten Verbindungen sind Gegenstand der vorliegenden Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise eine Verbindung der allgemeinen Formel II

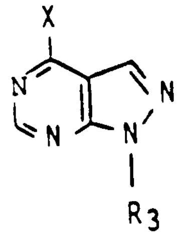

(II),

in welcher

X einen reaktiven Rest darstellt und

$R_3$ die obengenannte Bedeutung hat,

mit einer Verbindung der allgemeinen Formel III

$HNR_1R_2$ (III),

in welcher

$R_1$ und $R_2$ die obengenannte Bedeutung haben,

umsetzt und anschließend gewünschtenfalls

einen für $R_3$ stehen Rest durch einen anderen, durch die Definition von $R_3$ gegebenen Rest ersetzt

und die erhaltenen Verbindungen der Formel I gegebenenfalls durch Neutralisation mit nichttoxischen Säuren in ihre pharmakologisch verträglichen Salze umwandelt.

Als reakive Rest X kommen Chlor, Brom und Niederalkylthio in Frage.

Eine Umwandlung von Verbindungen der Formel I, in denen $R_3$ Wasserstoff bedeutet, in Verbindungen der Formel I, in denen $R_3$ verschiedenen von Wasserstoff ist, erfolgt zweckmäßig durch Alkylierung mit einer Verbindung $R_3$-Y, worin Y einen reaktiven Rest wie beispielsweise Halogen, Methansulfonyloxy oder Toluolsulfonyloxy bedeutet, in einem säurebindenden Milieu. Gegebenenfalls in $R_3$ vorhandene Hydroxysubstituenten können durch Ether-, Ester- oder Ketalgruppen geschützt und nach der Alkylierung freigelegt werden.

Für die Abspaltung eines für $R_3$ stehenden Tetrahydrofuranyloder Tetrahydropyranylrestes eignen sich anorganische Säuren wie Salzsäure oder Schwefelsäure in wässriger oder organischer Lösung.

Umgekehrt kann unter Protonenkatalyse in Verbindungen der Formel I, in denen $R_3$ Wasserstoff bedeutet, durch Umsetzung mit überschüssigem 2,3-Dihydrofuran bzw. 2,3-Dihydropyran der Tetrahydrofuranyl- bzw. Tetrahydropyranylrest eingeführt werden.

Die Ausgangsverbindungen der allgemeinen Formeln II und III sind literaturbekannte Substanzen oder können in Analogie zu literaturbekannten Verfahren hergestellt werden.

Die pharmakologisch verträglichen Salze erhält man in üblicher Weise, z.B. durch Neutralisation der Verbindungen der Formel I mit nichttoxischen anorganischen oder organischen Säuren wie z.B. Salzsäure, Schwefelsäure, Phosphorsäure, Bromwasserstoffsäure, Essigsäure, Milchsäure, Zitronensäure, Äpfelsäure, Salicylsäure, Malonsäure, Maleinsäure oder Bernsteinsäure.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäuren, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindusngsgemäßen Substanzen I auch in Form von Pudern und Salben verwendet werden. Sie werden dazu z.B. mit pulverförmigen, physiologisch verträglichen Verdünnungsmitteln bzw. üblichen Salbengrundlagen vermischt.

Die verabreichte Dosierung hängt vom Alter, der Gesundheit und dem Gewicht des Empfängers, dem

Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindungen 0.1 bis 50 mg/kg Körpergewicht. Normalerweise sind 0.5 bis 40 und vorzugsweise 1.0 bis 20 mg/kg Tag in einer oder mehreren Anwendungen pro Tag wirksam, um die gewünschten Resultate zu erhalten.

Besonders bevorzugt in Sinne der vorliegenden Erfindung sind außer den in den Beispielen genannten Verbindungen die folgenden:
4-<N-[1-(3-Brom-phenyl)ethyl]cyclopentylamino]-1H-pyrazolo[3,4-d]pyrimidin
4-<N-[2-(3-Brom-phenyl)ethyl]cyclopentylamino]-1H-pyrazolo[3,4-d]pyrimidin
4-[N-(3-Brom-pyridin-5-yl-methyl)cyclopentylamino]-1H-pyrazolo[3,4-d]pyrimidin
4-[N-(3-Ethoxycarbonyl-benzyl)cyclopentylamino]-1-(2,3-dihydroxy-propyl)-1H-pyrazolo[3,4-d]pyrimidin
4-[N-(3-Aminocarbonyl-benzyl)cyclopentylamino]-1-(2,3-dihydroxy-propyl)-1H-pyrazolo[3,4-d]pyrimidin
4-[N-(3-Cyan-benzyl)cyclopentylamino]-1-(2,3-dihydroxy-propyl)-1H-pyrazolo[3,4-d]pyrimidin
4-[N-(2,5-Dichlor-benzyl)cyclopentylamino]-1-(2,3-dihydroxy-propyl)-1H-pyrazolo[3,4-d]pyrimidin
4-[N-(3,4-Dichlor-benzyl)cyclopentylamino]-1-(2,3-dihydroxy-propyl)-1H-pyrazolo[3,4-d]pyrimidin
4-[N-(5-Chlor-2-methyl-benzyl)cyclopentylamino]-1-(2,3-dihydroxy-propyl)-1H-pyrazolo[3,4-d]pyrimidin
4-[N-(2-Chlor-5-methoxy-benzyl)cyclopentylamino]-1-(2,3-dihydroxy-propyl)-1H-pyrazolo[3,4-d]pyrimidin

## Beispiel 1

### 4-[N-(3-Chlor-benzyl)cyclopentylamino]-1H-pyrazolo-[3,4-d]pyrimidin

Eine Mischung aus 12.5 g (80 mmol) 4-Chlor-1H-pyrazolo[3,4-d]pyrimidin, 50.3 g (240 mmol) N-(3-Chlor-benzyl)-cyclopentylamin und 190 ml n-Butanol wird 16 h zum Rückfluß erhitzt. Man engt ein, nimmt den Rückstand in verd. Natronlauge auf, wäscht mit Ether, stellt die wässrige Phase mit Salzsäure neutral und filtriert den Niederschlag ab. Nach Umkristallisation aus Ethanol erhält man 19.5 g der Titelverbindung (74 % d. Th.) vom Schmp. 186-187 °C.

## Beispiel 2

In analoger Weise wie in Beispiel 1 beschrieben erhält man durch Umsetzung von 4-Chlor-1H-pyrazolo-[3,4-d]pyrimidin mit dem jeweiligen sekundären Amin:

| | Bezeichnung | Ausb. % | Schmelzpunkt °C (Lsg.mittel) |
|---|---|---|---|
| a) | 4-(N-Allyl-cyclohexylamino)-1H-pyrazolo[3,4-d]pyrimidin | 46 | 141-143 (Ethanol/Wasser) |
| b) | 4-[N-(3-Methyl-benzyl)cyclopentylamino]-1H-pyrazolo-[3,4-d]pyrimidin | 49 | 143-145 (Ethanol) |
| c) | 4-[N-(3-Trifluormethyl-benzyl)cyclopentylamino]-1H-pyrazolo[3,4-d]pyrimidin | 76 | 171-172 (Ethanol) |
| d) | 4-[N-(3-Methoxy-benzyl)cyclopentylamino]-1H-pyrazolo-[3,4-d]pyrimidin | 63 | 169-170 (Ethanol) |
| e) | 4-[N-(5-Chlor-2-methoxy-benzyl)cyclopentylamino]-1H-pyrazolo[3,4-d]pyrimidin | 40 | 232-235 (Dichlormethan/ Methanol) |
| f) | 4-[N-(3-Brom-benzyl)cyclopentylamino]-1H-pyrazolo-[3,4-d]pyrimidin | 55 | 192-194 (Ethylacetat) |
| g) | 4-[N-(3-Ethoxycarbonyl-benzyl)cyclopentylamino]-1H-pyrazolo[3,4-d]pyrimidin | 28 | 135-137 (Ethylacetat) |
| h) | 4-[N-(3-Aminocarbonyl-benzyl)cyclopentylamino]-1H-pyrazolo[3,4-d]pyrimidin | 25 | 150-160 amorph (Ether) |
| i) | 4-[N-(3-Cyan-benzyl)cyclopentylamino]-1H-pyrazolo-[3,4-d]pyrimidin | 52 | 178-180 (Ether) |

| Bezeichnung | | Ausb. % | Schmelzpunkt °C (Lsg.mittel) |
|---|---|---|---|
| j) | 4-[N-(2,5-Dichlor-benzyl)-cyclopentylamino]-1H-pyrazolo[3,4-d]pyrimidin | 32 | 233-235 (Ether) |
| k) | 4-[N-(3,4-Dichlor-benzyl)-cyclopentylamino]-1H-pyrazolo[3,4-d]pyrimidin | 27 | 168-170 (Ligroin) |
| l) | 4-[N-(5-Chlor-2-methyl-benzyl)cyclopentylamino]-1H-pyrazolo[3,4-d]pyrimidin | 33 | 203-205 (Ligroin) |
| m) | 4-[N-(2-Chlor-5-methoxy-benzyl)cyclopentylamino]-1H-pyrazolo[3,4-d]pyrimidin | 35 | 213-215 (Ether) |
| n) | 4-[N-(2,5-Dimethyl-benzyl)-cyclopentylamino]-1H-pyrazolo[3,4-d]pyrimidin | 41 | 188-190 (Ligroin) |
| o) | 4-[N-(2-Furyl-methyl)cyclopentylamino]-1H-pyrazolo-3,4-d]pyrimidin | 35 | 125-127 (Ethanol) |
| p) | 4-[N-(Thiophen-2-yl-methyl)-cyclopentylamino]-1H-pyrazolo[3,4-d]pyrimidin | 56 | 166-169 (Ligroin) |
| q) | 4-[N-(2-Methylpyridin-6-yl-methyl)cyclopentylamino]-1H-pyrazolo[3,4-d]pyrimidin | 79 | 182-184 (Ethanol) |

| | Bezeichnung | Ausb. % | Schmelzpunkt °C (Lsg.mittel) |
|---|---|---|---|
| r) | 4-[N-(3-Brom-benzyl)-2-propyl)amino]-1H-pyrazolo-[3,4-d]pyrimidin | 31 | 173-175 (Ether) |
| s) | 4-[N-(3-Brom-benzyl)-3-pentyl)amino]-1H-pyrazolo-[3,4-d]pyrimidin | 27 | 147-148 (Ether) |
| t) | 4-[N-(2-Brom-benzyl)cyclopentylamino]-1H-pyrazolo-[3,4-d]pyrimidin | 29 | 216-217 (Ether) |
| u) | 4-[N-(3-Brom-benzyl)cyclohexylamino]-1H-pyrazolo-[3,4-d]pyrimidin | 24 | 149-150 (Ether) |
| v) | 4-[N-(3-Brom-benzyl)methylamino]-1H-pyrazolo[3,4-d]-pyrimidin | 55 | 216-217 (Methanol) |
| w) | 4-[N-Allyl-(3-brom-benzyl)-amino]-1H-pyrazolo[3,4-d]-pyrimidin | 52 | 118-120 (Ethylacetat) |
| x) | 4-(N-Benzyl-cyclopentyl-amino)-1H-pyrazolo[3,4-d]-pyrimidin | 28 | 168-170 (Ether) |
| y) | 4-[N-(4-Brom-benzyl)cyclopentylamino]-1H-pyrazolo-[3,4-d]pyrimidin | 26 | 112-114 (Ether) |
| z) | 4-[N-(3-Iod-benzyl)cyclopentylamino]-1H-pyrazolo-[3,4-d]pyrimidin | 24 | 182-184 (Ether) |
| aa | 4-[N-(3-Hydroxy-benzyl)-cyclopentylamino]-1H-pyrazolo[3,4-d]pyrimidin | 43 | 213-215 (Ether) |
| ab | 4-[N-(3-Methylthio-benzyl)-cyclopentylamino]-1H-pyrazolo[3,4-d]pyrimidin | 48 | 116-118 (Ether) |
| ac | 4-[N-(3-t-Butyl-benzyl)-cyclopentylamino]-1H-pyrazolo[3,4-d]pyrimidin-hydrochlorid | 37 | 223-225 (Ethylacetat) |

| | Bezeichnung | Ausb. % | Schmelzpunkt °C (Lsg.mittel) |
|---|---|---|---|
| ad | 4-[N-(3,5-Dibrom-benzyl)-cyclopentylamino]-1H-pyrazolo-[3,4-d]pyrimidin | 17 | 182-184 (Ether) |
| ae | 4-[N-(3-Brom-4-methyl-benzyl)cyclopentylamino]-1H-pyrazolo-[3,4-d]pyrimidin | 19 | 200-202 (Ether) |
| af | 4-[N-(3-Brom-benzyl)anilino]-1H-pyrazolo-[3,4-d]pyrimidin | 40 | 194-196 (Methanol) |
| ag | 4-[N-(2-Brom-thiophen-5-yl-methyl)cyclopentylamino]-1H-pyrazolo-[3,4-d]pyrimidin | 33 | 155-156 (Ethylacetat) |

Beispiel 3

4-[N-(3-Chlor-benzyl)cyclopentylamino]-1-(2,3-dihydroxy-propyl)-1H-pyrazolo[3,4-d]pyrimidin

Zu einer Suspension von 1.5 g (30 mmol) 50proz. Natriumhydrid in 50 ml N,N-Dimethylformamid tropft man 9.6 g (30 mmol) 4-[N-(3-Chlor-benzyl)cyclopentylamino]-1H-pyrazolo[3,4-d]pyrimidin (Verbindung des Beispiels 1) in 25 ml N,N-Dimethylformamid, rührt 1 h bei 80 °C, fügt 10.0 g (35 mmol) 4-(4-Toluolsulfonyloxy-methyl)-2,2-dimethyl-1,3-dioxolan in 25 ml N,N-Dimethylformamid zu, rührt weitere 3 h bei 80 °C, engt im Vakuum ein, nimmt in Wasser auf, extrahiert mit Dichlormethan und engt den Extrakt ein. Man versetzt den Rückstand mit 300 ml 0.1 N Salzsäure, erhitzt 10 h zum Rückfluß, läßt abkühlen, wäscht mit Ether und stellt die wässrige Phase mit Natronlauge alkalisch.

Nach Extraktion mit Dichlormethan und Einengen des Extraktes verbleiben 8.2 g Rohprodukt, die durch Chromatographie an Kieselgel (Elutionsmittel Dichlormethan)/Methanol 9:1) gereinigt werden.

Man eluiert 4.1 g (34 % d.Th.) der Titelverbindung, die nach Verreiben mit Ether bei 98-100 °C schmelzen.

Beispiel 4

In analoger Weise wie in Beispiel 3 beschrieben erhält man durch Alkylierung mit 4-(4-Toluolsulfonyloxy-methyl)-2,2-dimethyl-1,3-dioxolan:

| Bezeichnung | | Ausb. % | Schmelzpunkt °C (Lsg.mittel) |
|---|---|---|---|
| a) | 4-(N-Allyl-cyclohexylamino)-1-(2,3-dihydroxy-propyl)-1H-pyrazolo[3,4-d]pyrimidin<br><br>aus Verbindung des Bsp. 2a) | 38 | Öl |
| b) | 4-[N-(3-Trifluormethyl-benzyl)cyclopentylamino]-1-(2,3-dihydroxy-propyl)-1H-pyrazolo[3,4-d]pyrimidin<br>aus Verbindung des Bsp. 2c | 33 | 122-123 (Ether) |
| c) | 4-[N-(2,5-Dimethyl-benzyl)-cyclopentylamino]-1-(2,3-dihydroxy-propyl)-1H-pyrazolo[3,4-d]pyrimidin<br><br>aus Verbindung des Besp. 2n) | 35 | 60-70 amorph (Dichlormethan/ Methanol) |
| d) | 4-[N-(2-Furyl-methyl)cyclopentylamino]-1-(2,3-dihydroxy-propyl)-1H-pyrazolo[3,4-d]pyrimidin<br><br>aus Verbindung des Bsp 2o) | 40 | Öl |

| | | | |
|---|---|---|---|
| e) | 4-[N-(Thiophen-2-yl-methyl)-cyclopentylamino]-1-(2,3-dihydroxy-propyl)-1H-pyrazolo-[3,4-d]pyrimidin aus Verbindung des Bsp. 2p) | 44 | Öl |
| f) | 4-[N-(3-Methoxy-benzyl)-cyclopentylamino]-1-(2,3-dihydroxy-propyl)-1H-pyrazolo-[3,4-d]pyrimidin aus Verbindung des Bsp. 2d) | 41 | Öl Hydrochlorid: 175-176 (Ethylacetat) |
| g) | 4-[N-(3-Methyl-benzyl)cyclo-pentylamino]-1-(2,3-di-hydroxy-propyl)-1H-pyrazolo-[3,4-d]pyrimidin | 65 | 80-82 (Ether) |
| h) | 4-[N-(3-Brom-benzyl)cyclo-pentylamino]-1-(2,3-di-hydroxy-propyl)-1H-pyrazolo-[3,4-d]pyrimidin | 51 | 107-108 (Ether) |
| i) | 4-[N-(5-Chlor-2-methoxy-benzyl)cyclopentylamino]-1-(2,3-dihydroxy-propyl)-1H-pyrazolo-[3,4-d]pyrimidin | 58 | 132-134 (Ether) |

Beispiel 5

4-[N-(2,5-Dimethyl-benzyl)cyclopentylamino]-1-(tetrahydrofuran-2-yl)-1H-pyrazolo[3,4-d]pyrimdin

Eine Mischung aus 7.8 g (35 mmol) 4-Chlor-1-(tetrahydrofuran-2-yl)-1H-pyrazolo[3,4-d]pyrimidin, 20.3 g (100 mmol) N-(2,5-Dimethyl-benzyl)cyclopentylamin und 100 ml n-Butanol wird 16 h zum Rückfluß erhitzt. Man engt ein, nimmt den Rückstand in Wasser auf, extrahiert mit Dichlormethan, wäscht den Extrakt mit verd. Essigsäure und verd. Natriumhydrogencarbonatlösung, trocknet, engt ein und chromatographiert an Kieselgel. Mit Dichlormethan/Methanol 98:2 eluiert man 5.0 g Titelverbindung (37 % d.Th.), die nach Verreiben mit Ether bei 173-174 °C schmelzen.

Beispiel 6

In analoger Weise wie in Beispiel 5 beschrieben erhält man durch Umsetzung von 4-Chlor-1-(tetrahydrofuran-2-yl)-1H-pyrazolo[3,4-d]pyrimidin mit dem jeweiligen sekundären Amin:

| | Bezeichnung | Ausb. % | Schmelzpunkt °C (Lsg.mittel) |
|---|---|---|---|
| a) | 4-(N-Allyl-cyclohexylamino)-1-(tetrahydrofuran-2-yl)-1H-pyrazolo[3,4-d]pyrimidin | 29 | Öl |
| b) | 4-[N-(2-Furyl-methyl)cyclopentylamino]-1-tetrahydrofuran-2-yl)-1H-pyrazolo-[3,4-d]pyrimidin | 33 | 85-87 (Ligroin/Ether) |
| c) | 4-[N-(Thiophen-2-yl-methyl)-cyclopentylamino]-1-tetrahydrofuran-2-yl)-1H-pyrazolo[3,4-d]pyrimidin | 46 | 120-122 (Ether) |
| d) | 4-[N-(3-Brom-benzyl)cyclopentylamino]-1-(tetrahydrofuran-2-yl)-1H-pyrazolo-[3,4-d]pyrimidin | 44 | 118-119 (Ligroin) |

## Patentansprüche

1. Pyrazolo [3,4-d]pyrimidine der Formel I

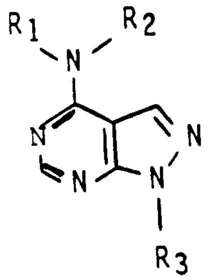

(I),

in welcher

$R_1$ einen $C_1$- bis $C_6$-Alkylrest, einen $C_2$- bis $C_6$-Alkenylrest, einen $C_3$- bis $C_7$-Cycloalkylrest oder einen Arylrest,

$R_2$ einen $C_2$- bis $C_6$-Alkenylrest, einen $C_3$- bis $C_7$-Cycloalkylrest oder einen gewünschtenfalls ein- oder mehrfach durch Halogen, $C_1$- bis $C_6$-Alkyl, Hydroxy, $C_1$- bis $C_6$-Alkoxy, $C_1$- bis $C_3$-Halogenalkyl, $C_3$- bis $C_7$-Alkoxycarbonyl, Aminocarbonyl, $C_2$- bis $C_7$-Alkylaminocarbonyl, $C_3$- bis $C_{13}$-Dialkylaminocarbonyl, Cyano oder $C_1$- bis $C_6$-Alkylthio substituierten Aralkyl-, Furylalkyl-, Thienylalkyl- oder Pyridinylalkylrest mit 1 bis 6 Kohlenstoffatomen im Alkylteil und

$R_3$ Wasserstoff, einen gewünschtenfalls ein- oder mehrfach durch Hydroxy substiuierten $C_2$- bis

$C_6$-Alkylrest, einen Tetrahydrofuranylrest oder einen Tetrahydropyranylrest bedeuten, mit der Maßgabe, daß $R_2$ nicht unsubstituiertes Benzyl sein kann, wenn $R_1$ für die Methylgruppe steht,
sowie deren physiologisch verträglichen Salze anorganischer oder organischer Säuren.

**2.** Pyrazolo[3,4-d]pyrimidine gemäß Anspruch 1, dadurch gekennzeichnet, daß $R_1$ einen $C_3$-$C_7$-Cycloalkylrest, insbesondere den Cyclopentyl- oder Cyclohexylrest bedeutet.

**3.** Pyrazolo[3,4-d]pyrimidine gemäß Anspruch 1, dadurch gekennzeichnet, daß $R_2$ einen gegebenenfalls ein- oder mehrfach durch Halogen, $C_1$-$C_6$-Alkyl, Hydroxy, $C_1$-$C_6$-Alkoxy, $C_1$-$C_3$-Halogenalkyl, $C_3$-$C_7$-Alkoxycarbonyl, Aminocarbonyl, $C_2$-$C_6$-Alkylaminocarbonyl, $C_3$-$C_{13}$-Dialkylaminocarbonyl, Cyano oder $C_1$-$C_6$-Alkylthio substituierten Ar-$C_1$-$C_6$-alkylrest bedeutet.

**4.** Pyrazolo[3,4-d]pyrimidine gemäß Anspruch 1 oder 3, dadurch gekennzeichnet, daß Aryl die Gruppen Phenyl und Naphthyl bedeutet.

**5.** Pyrazolo[3,4-d]pyrimidine gemäß Anspruch 1, dadurch gekennzeichnet, daß $R_3$ Wasserstoff, 2-Hydroxyethyl, 2-Hydroxypropyl, 3-Hydroxypropyl, 2,3-Dihydroxypropyl, Tetrahydrofuran-2-yl oder Tetrahydropyran-2-yl bedeutet.

**6.** Pyrazolo[3,4-d]pyrimidine gemäß Anspruch 1, ausgewählt aus der Gruppe der folgenden Verbindungen:
4-[N-(3-Methyl-benzyl)cyclopentylamino]-1H-pyrazolo[3,4-d]pyrimidin,
4-[N-(3-Trifluormethyl-benzyl)cyclopentylamino]-1H-pyrazolo[3,4-d]pyrimidin,
4-[N-(3-Methoxy-benzyl)cyclopentylamino]-1H-pyrazolo[3,4-d]pyrimidin,
4-[N-(3-Brom-benzyl)cyclopentylamino]-1H-pyrazolo[3,4-d]pyrimidin,
4-[N-(3-Methylthio-benzyl)-cyclopentylamino]-1H-pyrazolo[3,4-d]pyrimidin oder
4-[N-(2-Brom-thiophen-5-yl-methyl)cyclopentylamino]-1H-pyrazolo-[3,4-d]pyrimidin.

**7.** Verfahren zur Herstellung von Pyrazolo [3,4-d]-pyrimidine der Formel I gemäß den Ansprüchen 1-6, dadurch gekennzeichnet, daß man eine Verbindung der Formel II

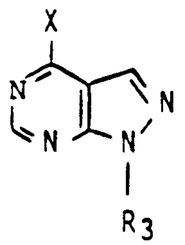

(II),

in welcher
X einen reaktiven Rest darstellt und
$R_3$ die obengenannte Bedeutung hat,
mit einer Verbindung der Formel III

$HNR_1R_2$ (III),

in welcher
$R_1$ und $R_2$ die obengenannte Bedeutung haben,
umsetzt und anschließend gewünschtenfalls
einen für $R_3$ stehen Rest durch einen anderen, durch die Definition von $R_3$ gegebenen Rest ersetzt
und die erhaltenen Verbindungen der Formel I gegebenenfalls durch Neutralisation mit nichttoxischen Säuren in ihre pharmakologisch verträglichen Salze umwandelt.

**8.** Arzneimittel, enthaltend eine Verbindung gemäß einem der Ansprüche 1-6 sowie übliche Träger- und Hilfsstoffe.

9. Verwendung von Verbindungen gemäß einem der Ansprüche 1-6 zur Herstellung von Arzneimitteln zur Behandlung allergischer Krankheiten sowie von entzündungsbedingten bronchospastischen und bronchokonstriktorischen Reaktionen.

## Claims

1. Pyrazolo[3,4-d]pyrimidines of the formula I

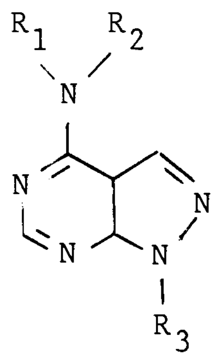

(I)

in which $R_1$ signifies a $C_1$ to $C_6$-alkyl radical, a $C_2$ to $C_6$-alkenyl radical, a $C_3$ to $C_7$-cycloalkyl radical or an aryl radical, $R_2$ a $C_2$ to $C_6$-alkenyl radical, a $C_3$ to $C_7$-cycloalkyl radical or an aralkyl, furylalkyl, thienylalkyl or pyrodinylalkyl radical, with 1 to 6 carbon atoms in the alkyl moiety substituted, if desired, one or more times by halogen, $C_1$ to $C_6$-alkyl, hydroxyl, $C_1$ to $C_6$-alkoxy, $C_1$ to $C_3$-haloalkyl, $C_3$ to $C_7$-alkoxycarbonyl, aminocarbonyl, $C_2$ to $C_7$-alkylaminocarbonyl, $C_3$ to $C_{13}$-dialkylaminocarbonyl, cyano or $C_1$ to $C_6$-alkylthio and $R_3$ hydrogen, a $C_2$ to $C_6$-alkyl radical substituted, if desired, one or more times by hydroxyl, a tetrahydrofuranyl radical or a tetrahydropyranyl radical, with the proviso that $R_2$ cannot be unsubstituted benzyl when $R_1$ stands for the methyl radical, as well as their physiologically acceptable salts of inorganic and organic acids.

2. Pyrazolo[3,4-d]pyrimidines according to claim 1, characterised in that $R_1$ signifies a $C_3$-$C_7$-cycloalkyl radical, especially the cyclopentyl or cyclohexyl radical.

3. Pyrazolo[3,4-d]pyrimidines according to claim 1, characterised in that $R_2$ signifies an Ar-$C_1$-$C_6$-alkyl radical possibly substituted one or more times by halogen, $C_1$-$C_6$-alkyl, hydroxyl, $C_1$-$C_6$-alkoxy, $C_1$-$C_3$-haloalkyl, $C_3$-$C_7$-alkoxycarbonyl, aminocarbonyl, $C_2$-$C_6$-alkylaminocarbonyl, $C_3$-$C_{13}$-dialkylaminocarbonyl, cyano or $C_1$-$C_6$-alkylthio.

4. Pyrazolo[3,4-d]pyrimidines according to claim 1 or 3, characterised in that aryl signifies the groups phenyl and naphthyl.

5. Pyrazolo[3,4-d]pyrimidines according to claim 1, characterised in that $R_3$ signifies hydrogen, 2-hydroxyethyl, 2-hydroxypropyl, 3-hydroxypropyl, 2,3-dihydroxypropyl, tetrahydrofuran-2-yl or tetrahydropyran-2-yl.

6. Pyrazolo[3,4-d]pyrimidines according to claim 1, selected from the group of the following compounds:
4-[N-(3-methylbenzyl)-cyclopentylamino]-1H-pyrazolo[3,4-d]pyrimidine,
4-[N-(3-trifluoromethylbenzyl)-cyclopentylamino]-1H-pyrazolo[3,4-d]pyrimidine,
4-[N-(3-methoxybenzyl)-cyclopentylamino]-1H-pyrazolo[3,4-d]pyrimidine,
4-[N-(3-bromobenzyl)-cyclopentylamino]-1H-pyrazolo[3,4-d]pyrimidine,
4-[N-(3-methylthiobenzyl)-cyclopentylamino]-1H-pyrazolo[3,4-d]pyrimidine or
4-[N-(2-bromothiophen-5-ylmethyl)-cyclopentylamino]-1H-pyrazolo[3,4-d]pyrimidine.

7. Process for the preparation of pyrazolo[3,4-d]pyrimidines of the formula I according to claims 1 - 6, characterised in that one reacts a compound of the formula II

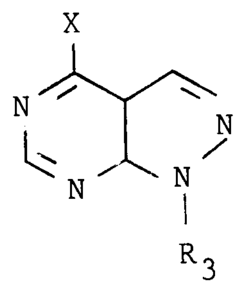

in which X represents a reactive residue and $R_3$ has the above-mentioned meaning, with a compound of the formula III

$HNR_1R_2$ (III)

in which $R_1$ and $R_2$ have the above-mentioned meanings, and subsequently, if desired, replaces a radical standing for $R_3$ by the radical given by the definition of $R_3$ and possibly converts the compounds obtained of the formula I into their pharmacologically acceptable salts by neutralisation with non-toxic acids.

**8.** Medicaments containing a compound according to one of claims 1 - 6, as well as usual carrier and adjuvant materials.

**9.** Use of compounds according to one of claims 1 - 6 for the preparation of medicaments for the treatment of allergic diseases, as well as of inflammation-caused bronchospastic and bronchoconstrictory reactions.

**Revendications**

**1.** Pyrazolo[3,4-d]pyrimidine de formule I

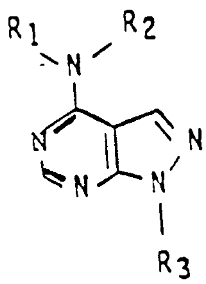

(I),

dans laquelle

$R_1$ représente un groupe alkyle en $C_1$-$C_6$, un groupe alcényle en $C_2$-$C_6$, un groupe cycloalkyle en $C_3$-$C_7$ ou un groupe aryle,

$R_2$ représente un groupe alcényle en $C_2$-$C_6$, un groupe cycloalkyle en $C_3$-$C_7$ ou un groupe aralkyle éventuellement substitué une ou plusieurs fois par un atome d'halogène, un groupe alkyle en $C_1$-$C_6$, un groupe hydroxy, un groupe alcoxy en $C_1$-$C_6$, un groupe halogénoalkyle en $C_1$-$C_3$, un groupe alcoxycarbonyle en $C_3$-$C_7$, un groupe aminocarbonyle, un groupe alkyl-($C_2$-$C_7$)aminocarbonyle, un groupe dialkyl($C_3$-$C_{13}$)aminocarbonyle, un groupe cyano ou un groupe alkyl($C_1$-$C_6$)thio, un groupe furylalkyle, un groupe thiénylalkyle ou un groupe pyridinylalkyle, avec 1 à 6 atomes de carbone dans la partie alkyle, et

$R_3$ représente un atome d'hydrogène, un groupe alkyle en $C_2$-$C_6$ éventuellement substitué une à plusieurs fois par un groupe hydroxy, un groupe tétrahydrofuranyle ou un groupe tétrahydropyranyle,

étant entendu que R ne peut représenter un groupe benzyle non substitué lorsque $R_1$ représente un groupe méthyle,

ainsi que leurs sels physiologiquement acceptables, formés avec des acides minéraux ou organiques.

**2.** Pyrazolo[3,4-d]pyrimidine selon la revendication 1, caractérisée en ce que $R_1$ représente un groupe cycloalkyle en $C_3$-$C_7$, en particulier un groupe cyclopentyle ou cyclohexyle.

**3.** Pyrazolo[3,4-d]pyrimidine selon la revendication 1, caractérisée en ce que $R_2$ représente un groupe Aralkyle($C_1$-$C_6$), éventuellement substitué une ou plusieurs fois par un atome d'halogène, un groupe alkyle en $C_1$-$C_6$, hydroxy, alcoxy en $C_1$-$C_6$, halogénoalkyle en $C_1$-$C_3$, alcoxy($C_3$-$C_7$)carbonyle, aminocarbonyle, alkyle($C_1$-$C_6$)aminocarbonyle, dialkyl($C_3$-$C_{13}$)aminocarbonyle, cyano ou alkyl($C_1$-$C_6$)thio.

**4.** Pyrazolo[3,4-d]pyrimidine selon la revendication 1 ou 3, caractérisée en ce que le groupe aryle représente les groupes phényle et naphtyle.

**5.** Pyrazolo[3,4-d]pyrimidine selon la revendication 1, caractérisée en ce que $R_3$ représente un atome d'hydrogène, les groupes 2-hydroxyéthyle, 2-hydroxypropyle, 3-hydroxypropyle, 2,3-dihydroxypropyle, tétrahydrofuran-2-yle ou tétrahydropyran-2-yle.

**6.** Pyrazolo[3,4-d]pyrimidine selon la revendication 1, choisie dans le groupe constitué par les composés suivants :
4-[N-(3-méthyl-benzyl)cyclopentylamino]-1H-pyrazolo[3,4-d]pyrimidine,
4-(N-(3-trifluorométhyl-benzyl)cyclopentylamino]-1H-pyrazolo-[3,4-d]pyrimidine,
4-[N-(3-méthoxy-benzyl)cyclopentylamino]-1H-pyrazolo[3,4-d]pyrimidine,
4-[N-(3-bromo-benzyl)cyclopentylamino]-1H-pyrazolo[3,4-d]pyrimidine,
4-[N-(3-méthylthio-benzyl)cyclopentylamino]-1H-pyrazolo[3,4-d]pyrimidine ou
4-[N-(2-bromo-thiophène-5-yl-méthyl)cyclopentylamino]-1H-pyrazolo-[3,4-d]pyrimidine.

**7.** Procédé pour la préparation de la pyrazolo[3,4-d]pyrimidine de formule I selon les revendications 1 à 6, caractérisé en ce qu'on fait réagir un composé de formule II

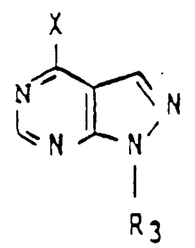

(II),

dans laquelle
X représente un groupe réactif et
$R_3$ a la signification mentionnée ci-dessus,
avec un composé de formule III,

$HNR_1R_2$ (III),

dans laquelle
$R_1$ et $R_2$ ont les significations mentionnées ci-dessus, et ensuite, on remplace éventuellement un groupe représenté par $R_3$ par un autre groupe répondant à la définition de $R_3$, et on transforme les composés de formule I ainsi obtenus, éventuellement par neutralisation avec des acides non toxiques, en leurs sels pharmacologiquement acceptables.

**8.** Médicament contenant un composé selon l'une quelconque des revendications 1 à 6, ainsi que des véhicules et adjuvants usuels.

**9.** Utilisation des composés selon l'une quelconque des revendications 1 à 6 pour la préparation de médicaments pour le traitement d'affections allergiques, ainsi que de réactions bronchospastiques et bronchoconstrictrices d'origine inflammatoire.